# EUROPEAN PATENT SPECIFICATION

(11) **EP 3 083 196 B1**
(45) Date of publication and mention of the grant of the patent: **17.04.2019**
(21) Application number: 14825057.4
(22) Date of filing: 19.12.2014
(51) Int. Cl.: B29C 65/56, A61F 13/15, B31D 1/04, B31F 1/07

(54) **BONDING APPARATUS**
VERBINDUNGSVORRICHTUNG
APPAREIL DE LIAISON

(30) Priority: 20.12.2013 US 201314135687
(43) Date of publication of application: 26.10.2016
(73) Proprietor: The Procter & Gamble Company, Cincinnati, OH 45202 (US)
(72) Inventor: POWELL, Craig, Allen, Cincinnati, Ohio 45202 (US); COE, Richard, George, Cincinnati, Ohio 45202 (US); WADMAN, Richard, James, Cincinnati, Ohio45202 (US); DENG, Rong, Cincinnati, Ohio 45202 (US)
(74) Representative: Kremer, Véronique Marie Joséphine
(86) International application number: PCT/US2014/071412
(87) International publication number: WO 2015/095662

(56) References cited:
- US-A1- 2004 116 889
- US-A1- 2007 240 586

## Description

### FIELD OF THE INVENTION

The present disclosure relates to methods for manufacturing absorbent articles, and more particularly, to apparatuses and methods for bonding substrates that may be used as components of absorbent articles.

### BACKGROUND OF THE INVENTION

Along an assembly line, various types of articles, for example sanitary napkins, diapers, and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from advancing web or webs are combined with other individual components created from other advancing web or webs. Webs of material and component parts used to manufacture diapers may include: backsheets, topsheets, leg cuffs, waist bands, absorbent core components, front and/or back ears, fastening components, and various types of elastic webs and components such as leg elastics, barrier leg cuff elastics, stretch side panels, and waist elastics. Webs of material and component parts used to manufacture sanitary napkins may include: backsheets, topsheets, secondary topsheets, absorbent core components, release paper wrappers, and the like. Once the desired component parts are assembled, the advancing web(s) and component parts are subjected to a final knife cut to separate the web(s) into discrete articles.

During the assembly process, various components and/or advancing webs of material may be bonded together in various ways. For example, in some processes, advancing webs and/or components may be bonded together with adhesives. In other processes, advancing webs and/or components may be mechanically bonded together with heat and/or pressure without the use of adhesives. An example of such a mechanical bonding method and apparatus is disclosed in U.S. Patent No. 4,854,984, wherein two laminae are bonded together by advancing through a nip between a patterned cylinder and an anvil cylinder. Pattern elements on the patterned cylinder exert pressure on the two laminae against the anvil roll to create discrete bond sites. More particularly, bond sites are created as the extreme nip pressure compresses and yields the laminae material in areas between the pattern elements and the anvil. During the bonding process, some of the yielded material may flow from the bond site to areas surrounding the perimeter of the pattern element.

These mechanical bonding methods may damage the resultant laminate web by forming holes and/or tears in or around the bond sites. For example, pattern elements may comprise sharp edges and may tear, cut, or weaken the bonded web in areas adjacent to the bonds. Tears may propagate from one bond site to another, causing a zippering of the web. This often creates a consumer-noticeable defective product. In addition, as the web basis weight of the laminate decreases, bonds may become more susceptible to bond defects such as tearing and pinholes at relatively high nip pressures.

US 2004/116889A1 (Carbone, et al.) discloses a mechanical fastening system for an article wherein a first fastening component is secured to the article within an attachment region. A portion of the first fastening component within the attachment region is mechanically bonded to the article, with a ratio of an area of the bonded portion to an area of the attachment region defining a percent bonded area of the first fastening component. A second fastening component is secured to the article within an attachment region thereof. A portion of the second fastening component within the attachment region is mechanically bonded to the article, with a ratio of an area of the bonded portion to an area of the attachment region thereof defining a percent bonded area of the second fastening component. The percent bonded area of the first fastening component is substantially less than the percent bonded area of the second fastening component.

Consequently, it would be beneficial to provide a method and apparatus for mechanically bonding substrates that produces bond sites with relatively low damage to the laminae. Previous attempts to address these problems are not desirable due to cost and complexity. For instance, a thermal energy mechanical bonding method and apparatus is disclosed in U.S. Patent No. 7,971,526 wherein facetted impression elements comprise at least two chamfered surfaces. There is a need for a nub with only one chamfered or radiused surface such that it is cheaper and easier to make. There is a desire for bonding nubs which minimize the applied process strain on a substrate, so that this applied process strain is less than the failure strain of the substrate of interest. There is a need to reduce or eliminate substrate tearing as a result of bonding.

### SUMMARY OF THE INVENTION

The present disclosure relates to methods and apparatuses for mechanically bonding substrates together. A bonding roll may be adjacent an anvil roll to define a nip between the bonding surfaces and the anvil roll, wherein the bonding roll is biased toward the anvil roll to define a nip pressure between bonding surfaces and the anvil roll. As substrates advance between the bonding roll and anvil roll, the substrates are compressed between the anvil roll and the bonding surfaces to form a discrete bond region between the substrates.

The invention is defined by an apparatus according to claim 1.

### BRIEF DESCRIPTION OF THE DRAWINGS

The following detailed description can be best understood when read in conjunction with the drawings enclosed herewith.
Figure 1 is a schematic side view of a bonding apparatus.
Figure 2 is a perspective view of the bonding apparatus of Figure 1, showing a bonding roll and an anvil roll.
Figure 3A is an enlarged perspective view of an exemplary chamfered nub.
Figure 3B is a top view of the chamfered nub of Figure 3A.
Figure 3C is a side view of the chamfered nub of Figure 3A.
Figure 3D is an enlarged view of a portion of the chamfered nub of Figure 3C.
Figure 4A is an enlarged perspective view of an exemplary radiused nub.
Figure 4B is a top view of the radiused nub of Figure 4A.
Figure 4C is a side view of the radiused nub of Figure 4A.
Figure 5 illustrates a process for making an absorbent article, wherein the process comprises the bonding apparatus of Figure 2.
Figure 6 is a top view of an absorbent article.
Figure 7 is a cross-sectional view of the absorbent article taken about line 2-2 of Figure 6.
Figure 8 is an exploded view of the absorbent article cross section of Figure 7.

### DETAILED DESCRIPTION OF THE INVENTION

### Definitions

The following term explanations may be useful in understanding the present disclosure:
As used herein, the term "absorbent article" includes disposable articles such as sanitary napkins, panty liners, tampons, interlabial devices, wound dressings, diapers, adult incontinence articles, wipes, and the like. At least some of such absorbent articles are intended for the absorption of body liquids, such as menses or blood, vaginal discharges, urine, and feces. Wipes may be used to absorb body liquids, or may be used for other purposes, such as for cleaning surfaces. Various absorbent articles described above will typically comprise a liquid pervious topsheet, a liquid impervious backsheet joined to the topsheet, and an absorbent core between the topsheet and backsheet.

As used herein, the term "nub" refers to any element on the surface of a roll that is capable of bonding two or more substrates.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together (a "composite substrate"). As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The term "yield" is used herein to refer to permanent and non-reversible material displacement due to subjecting the material to mechanical stress past the yield stress of the material and/or permanent and non-reversible material displacement due to subjecting the material to temperatures higher than the melting point of the material.

### Bonding Apparatus

The present disclosure relates to methods and apparatuses for manufacturing absorbent articles, and in particular, to methods and apparatuses for mechanically bonding substrates together. The apparatuses may include a bonding roll and an anvil roll. The bonding roll may include a plurality of bonding elements, or nubs, protruding radially outward, wherein each nub includes a bonding surface. And the bonding roll may be adjacent the anvil roll to define a nip between the bonding surfaces and the anvil roll, wherein the bonding roll is biased toward the anvil roll to define a nip pressure between bonding surfaces and the anvil roll. As the first and second substrates advance between the bonding roll and anvil roll, the first substrate and the second substrate are compressed between the anvil roll and the bonding surfaces to form a discrete bond region between the first and second substrates. More particularly, during the bonding process, the nip pressure and/or heat generated by the nip pressure causes the first and second substrate material to yield. And the yielded material is pressed together to form a bond region. In addition, some of the yielded material flows outward from under the bonding surfaces to form one or more outer grommet regions along the outer perimeter of one or more nubs. As discussed in more detail below, the bonding surfaces can also be separated from each other by a controlled gap where bonding pressure is created by the compressive stiffness of the materials. During this gap bonding process, bearer rings may be used to control the gap between the bonding and anvil rolls.

It is to be appreciated that various arrangements and configurations of the apparatuses and methods herein may be used to bond various types of substrates together. For example, as discussed in more detail below, apparatuses and methods according to the present disclosure may be utilized to bond various substrates together during the production of various components of absorbent articles, such as sanitary napkins or incontinence articles.

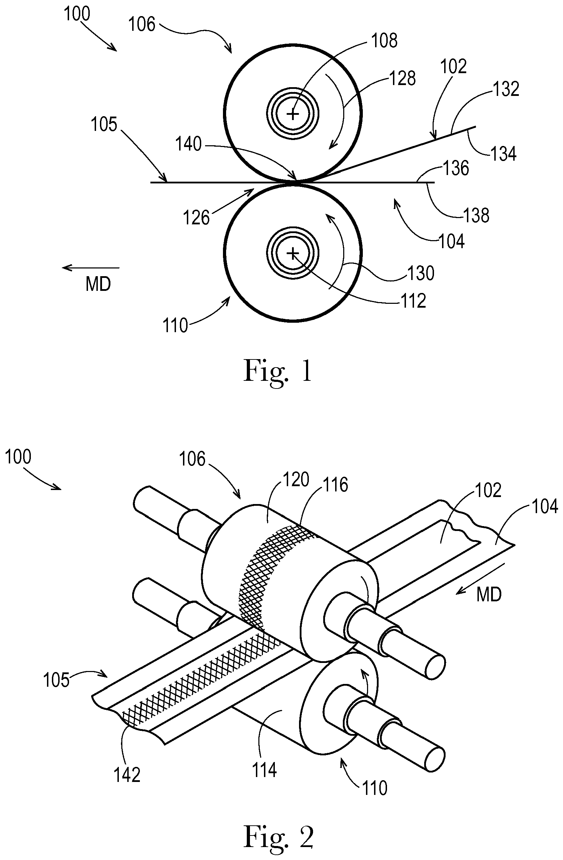

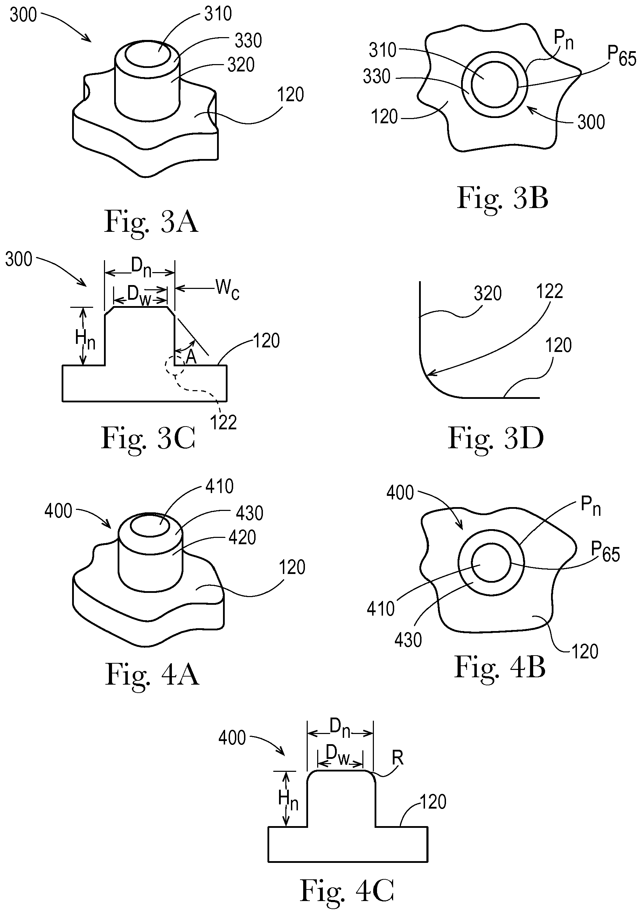

Figures 1 and 2 show an embodiment of a bonding apparatus 100 that may be used to bond a first substrate 102 and a second substrate 104 together to form a laminate 105. The bonding apparatus 100 may include a bonding roll 106 adapted to rotate around an axis of rotation 108, and an anvil roll 110 adapted to rotate around an axis of rotation 112. As shown in Figure 2, the anvil roll 110 includes an outer circumferential surface 114 which is preferably smooth, and the bonding roll 106 includes one or more bonding elements, or nubs, 116. The bonding roll 106 is adjacent the anvil roll 110 so as to define a nip 126 between the bonding roll 106 and the anvil roll 110, and more particularly, to define a nip 126 between the bonding surface (310, 410 shown in Figures 3 and 4) of each nub 116 and the anvil roll 110. It is to be appreciated that the bonding roll 106 and the anvil roll 110 may be configured to rotate such that the bonding surfaces on the bonding roll 106 and the outer circumferential surface 114 of the anvil roll 110 move at the same speeds or different speeds.

During the bonding operation, the bonding roll 106 may rotate in a first direction 128 around the axis of rotation 108 of the bonding roll 106, and the anvil roll 110 may rotate in a second direction 130, opposite the first direction 128, around the axis of rotation 112 of the anvil roll 110. The first substrate 102 and second substrate 104 may advance in a machine direction MD between the bonding roll 106 and the anvil roll 110. More particularly, the first substrate 102 includes a first surface 132 and a second surface 134 opposite the first surface 132, and the second substrate 104 includes a first surface 136 and a second surface 138 opposite the first surface 136. As such, the first surface 132 of the first substrate 102 is contacted by the bonding roll 106, and the second surface 138 of the second substrate 104 is contacted by the anvil roll 110. And the second surface 134 of the first substrate 102 and the first surface 136 of the second substrate 104 contact each other. As first substrate 102 and second substrate 104 advance through the nip 126 between the bonding surface of a nub 116 and the anvil roll 110, the nub 116 contacts the first substrate 102 and compresses the first substrate 102 and second substrate 104 between the bonding surface of the nub 116 and the anvil roll 110. In turn, heat generated by the nip pressure causes the first and second substrate material to yield. The bonding surface 118 presses yielded material 140 of the first and second substrates 102, 104 together to form a discrete bond region 142 between the first and second substrates 102, 104. Thus, the apparatus 100 may form a laminate 105 including first and second substrates 102, 104 bonded together by discrete bond regions 142, without the use of adhesives. It is to be appreciated, however, that the bonding apparatus 100 may also be used in combination with adhesives. Although Figure 1 shows the apparatus 100 bonding two substrates together, it is to be appreciated that the apparatus may bond more than two substrates together. In addition, it is to be appreciated that the apparatus may also be used to bond fibers of nonwoven together on a single substrate. The anvil roll and bonding roll are not heated. In one embodiment, the anvil roll is cooled to less than 50°C, or optimally below 30°C. This can be accomplished via, for instance, glycol-water cooling. When adhesives are included in combination, the rolls can be heated or cooled to reduce buildup.

### Bonding Nubs

Figures 3A-3D depict preferred bonding nub geometries, while figures 4A-4C depict bonding nub geometries that do not form part of the invention. Each nub 300, 400 is generally cylindrical and includes a bonding surface 310, 410, a circumferential sidewall 320, 420, and a shoulder (or edge break) 330, 340 located between the sidewall and the bonding surface. Bonding roll 106 includes a base circumferential surface 120, from which each sidewall 320, 420 protrudes radially outward to define a nub height Hn between the bonding surface 310, 410 and the base surface 120. The nub height is from 1mm to 3mm, or 1.5mm to 2mm. The nubs 300, 400 may be configured with the same or different heights. In addition, the nub height may be greater than the sum of the thicknesses of the substrates 102, 104 being bonded. For taller nubs, the chamfer results in a perimeter-wrapped length around the nub such that the strain is less than the breaking strain of the substrate. Nub heights of less than 1mm are generally undesirable for use with thicker substrates. Taller nubs are desired so that there is no load sharing by the substrate in regions between the nubs.

Sidewall 320, 420 is substantially perpendicular to base surface 120. Sidewall 320, 420 is at a 90 degree angle-plus or minus 5 degrees, or plus or minus 3 degrees, or plus or minus 1 degree-measured from the base surface 120. The sidewall is substantially straight (non-tapered). The nub 300 may comprise a curvilinear portion, or root radius 122, of about 0.5mm located between the sidewall 320 and the base surface 120. Accordingly, the sidewall 320 may not form a sharp angle with the base surface 120. Shoulder 330 is chamfered (linear) while shoulder 430 is radiused (curvilinear). When the nub shoulder is chamfered, the chamfer angle A is from 50 to 60 degrees, or about 55 degrees. The chamfered nub has only one angle at the shoulder. The chamfer width Wc (or, the width of the shoulder), measured in a plane parallel to the base surface 120, is from 0.2mm to 0.6mm, from 0.25mm to 0.5mm, or about 0.3mm. When the nub is radiused, the radius R is from 0.2mm to 0.8mm, from 0.3mm to 0.5mm, or about 0.4mm. The nub diameter Dn is from 2mm to 5mm, or from 2mm to 3 mm. The bonding surface diameter Dbs is from 1mm to 3mm, or 1.5mm to 2mm. A ratio of the bonding surface diameter Dbs to the nub diameter Dn is 0.75 or less.

The circumferential sidewall 320, 420 defines an outer perimeter Pn of the nub 300, 400. The nubs may have a perimeter that defines circular, square, rectangular, and various types of other shapes. For example, the nubs may have a perimeter that defines an elliptical shape. As such, in some embodiments, an elliptically shaped nub may have a major axis of about 1.27 mm and minor axis of about 0.56 mm. In some instances, the nubs may be configured such that resulting bond regions also offer aesthetic benefits such as, for example, a stitched-like appearance along with a relatively smooth texture feel to the skin. The bonding surface 310, 410 comprises a perimeter Pbs. The radius or chamfer on the nub shoulder may vary around the perimeter. In the nubs shown, the bonding surface perimeter and the nub perimeter are concentric circles. However, it is possible to create a nub wherein the perimeters are different shapes (*e*.*g*., one circular and one elliptical) or non-concentric.

It is to be appreciated that various nub geometries may be used with the bonding apparatuses and processes herein. Nubs may be round, oval, ellipse, polygonal, or combinations thereof. Various quantities of nubs may be arranged in groupings, or patterns, to form discrete bonds (rather than long channels, for example, which are often found with embossing). Spacing between nubs may be 1mm to 20mm, 1mm to 5mm, or 1mm to 2mm.

Nubs may be made via jig grinding, electrical discharge machining, or the like. It is generally cheaper and faster to manufacture chamfered nubs vs. radiused nubs; in addition, tighter tolerances can be achieved with chamfered nubs.

It is to be appreciated that the bonding apparatus 100 may also be configured in various ways. For example, different types of motor arrangements may be used to rotate the bonding roll 106 and anvil roll 110. For example, the bonding roll 106 and the anvil roll 110 may be driven independently with two independent motors. Or, a motor may be used to directly drive the bonding roll and via pulley and belt drive the anvil roll. Or, when bearer rings are used, only one of the rolls may be driven, and the other roll is driven by the contact surfaces of the bearer rings. In addition, the nip pressure between the bonding surface and the anvil roll may be generated in various ways. In some embodiments, the bonding apparatus 100 is configured to define a nip pressure above 345 MPa (50,000 PSI) between the bonding surface 118 and the anvil roll 110. In some embodiments, the bonding apparatus 100 is configured to define a nip pressure from about 276 MPa (40,000 PSI) to about 827 MPa (120,000 PSI) or from about 414 MPa (60,000 PSI) to about 483 MPa (70,000 PSI) between the bonding surface 118 and the anvil roll 110. In some embodiments, the bonding apparatus 100 is configured to define a nip pressure of about 414 MPa (60,000 PSI) between the bonding surface 118 and the anvil roll 110.

Nubs with significant shoulders, or edge breaks, have been proven to improve bond strength and reduce the tendency of a substrate, e.g., a topsheet, to peel. It is believed that the improved nubs create stronger bonds in part due to less torn fibers/filaments next to the fusion bond sites. It is desirable to use a nub where the strain induced by the perimeter distance of the web wrapped around the nub is below the failure strain (strain at maximum force) of all materials. The reduced shearing or tearing of fibers is believed to be the cause of improved fusion bond strength, which results in greater peel force required to delaminate, for example, the topsheet from the secondary topsheet. In addition, such nubs with a significant chamfer or radius provide for a gradient in the squeeze flow of yielded material from underneath the nub, thus reducing the stress concentrations at the periphery of the bond. Traditional nubs have very small chamfers or radii edge breaks, and appear to shear more fibers at the edge of the fusion bond, resulting in lower peel force required to delaminate the topsheet from the secondary topsheet of a feminine hygiene article.

The process can comprise the steps of layering a cellulosic airlaid web and a film topsheet (and, optionally a spunlace or other intermediate layer) and applying a pressure to bond the layers together wherein the process uses a smooth anvil roll and a bonding roll containing the nubs with the prescribed chamfers or radii curvature on the top edge breaks of the nubs. The bonding roll can have a combination of chamfers and radii such that the deformed web path perimeter is less than the failure strain of the materials.

### General Description of a Process Incorporating the Bonding Apparatus

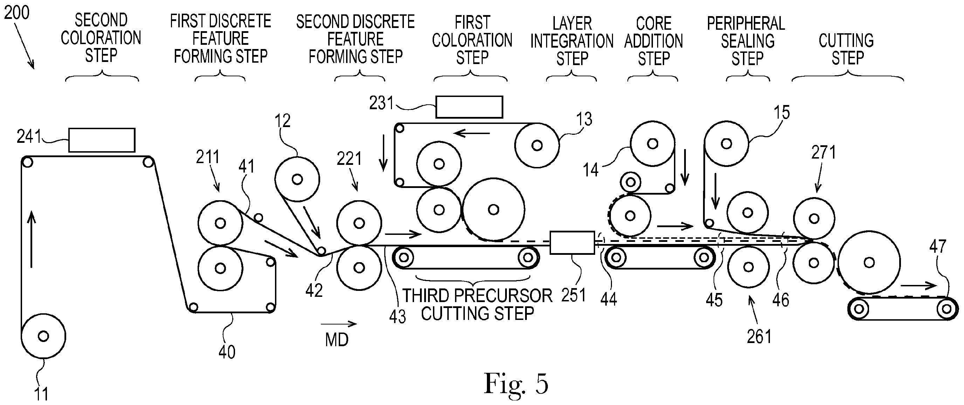

As previously mentioned, the bonding apparatus and method according to the present disclosure may be utilized to assemble various components of absorbent articles such as sanitary napkins or incontinence articles. For example, Figure 5 shows a schematic view of a converting apparatus 200 adapted to manufacture absorbent articles. In the process carried out in Figure 2, initially a polymer film 11 is produced with a second colored region in a second coloration unit 241. The second colored region can be provided on either side of the polymer film 11. Alternatively, the second colored region can be provided in a nonwoven 12. When the polymer film 11 or the nonwoven 12 already has a second colored region before conducting the second coloration step, the second coloration step may be skipped, or still employed to provide additional colored region on the polymer film 11 or the nonwoven 12.

A colored polymer film 40 is fed into a first discrete feature forming unit 211 to form a deformed polymer film 41. Then, the nonwoven 12 is supplied onto the deformed polymer film 41 to form a layered composite 42, and the layered composite 42 is fed into a second discrete feature forming unit 221 to form a deformed layered composite 43. In this example, the first and second discrete feature forming units 211, 221 may comprise two generally cylindrical rollers wherein at least one of the two rollers in each unit has discrete feature forming elements on its surface. A step of forming the layered composite 42 and a step of forming the second discrete features can be carried out sequentially as illustrated, or it can be carried out simultaneously. Then, the precursor sheet 13 is supplied onto a nonwoven side of the deformed layered composite 43. The precursor sheet 13, before being supplied onto the deformed layered composite 43 to form an integrated layered composite 44, is provided with a first colored region in a first coloration unit 231, and may be cut into a predetermined size and shape, then is supplied onto a nonwoven side of the deformed layered composite 43. When the precursor sheet 13 already has a first colored region before conducting the first coloration step, the first coloration step may be skipped, or still employed to provide additional colored region on the precursor sheet 13. Then, the deformed layered composite 43 and the precursor sheet 13 are integrated at a bonding unit 251 to form an integrated layered composite 44. An absorbent core 14, which can be a continuous sheet or in a determined size and shape, is supplied onto a precursor sheet side of the integrated layered composite 44 to form an absorbent layered composite 45. A precursor backsheet 15 is supplied and adhered onto an absorbent core side of the absorbent layered composite 45 to provide peripheral seal in a peripheral seal unit 261 along a peripheral line of an absorbent article and to form an absorbent article assembly 46. The absorbent article assembly 46 is then cut by a cutting unit 271 into individual absorbent articles 47. In one embodiment, the line speed of the converting apparatus is at least about 366 m/min (1200 ft/min).

### General Description of an Absorbent Article

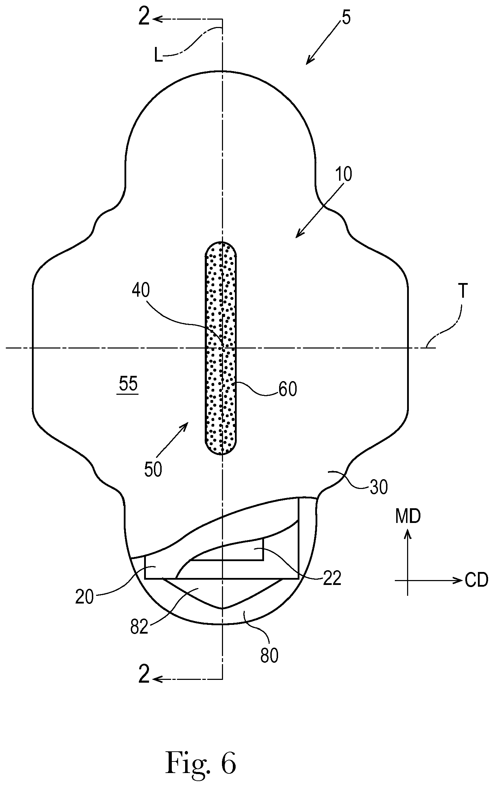

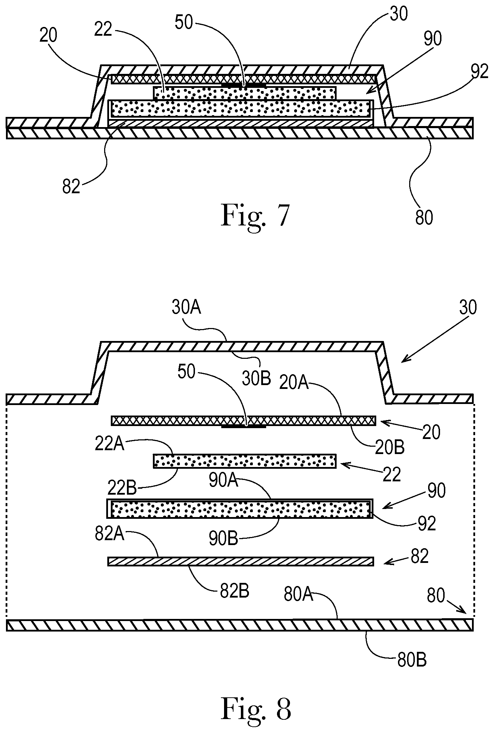

An example absorbent article 5 according to the present disclosure, shown in the form of a sanitary napkin or incontinence pad, is represented in Figures 6-8. This type of absorbent article is shown for illustration purpose only as the present disclosure can be used for making a wide variety of other absorbent articles. Figure 6 is a top view of the example absorbent article 5, in a flat-out state, with portions of the structure being cut-away to more clearly show the construction of the absorbent article 5. Figure 7 is a cross-sectional view of the absorbent article of Figure 1 taken along line 2-2, while Figure 8 is an exploded cross-sectional view of the absorbent article of Figure 7.

Referring to Figure 6, the absorbent article 5 can have a substantially planar configuration and a centroid 35. The centroid 35 is the in-plane center of mass of the absorbent article 5. The centroid 35 is at the intersection between the longitudinal centerline L and transverse centerline T. The transverse centerline T is orthogonal to the longitudinal centerline L. The absorbent article 5 can, but need not be, symmetric about the transverse centerline T. The absorbent article 5 has a body-facing surface 10 and a garment facing surface (not shown).

The absorbent article 5 comprises a plurality of layers to promote certain liquid handling behaviors. Example layers include a liquid-permeable topsheet 30 and an absorbent core 90. Some embodiments can also include a top core 22, as illustrated. The absorbent core 90 can have a number of suitable arrangements, for example the absorbent core 90 can have a tissue outer wrapping 92 (Figure 8). The absorbent articles can also have a backing material 82 and a backsheet 80.

To help ensure that fluids flow into the absorbent core 90, some absorbent articles are constructed with what is sometimes referred to as a secondary topsheet 20 ("STS") positioned intermediate the topsheet 30 and the absorbent core 90. This secondary topsheet 20 is designed to acquire the fluid on the liquid-permeable topsheet 30 and distribute it to the underlying absorbent core 90. To help ensure that the secondary topsheet 20 transfers the fluid to the absorbent core 90, the secondary topsheet 20 can have sufficient capillarity to draw the fluid through the liquid-permeable topsheet 30. To ensure that the fluid flow continues onto the absorbent core 90, the secondary topsheet 20 can be designed with more permeability than the absorbent core 90, and less capillarity than the absorbent core 90. For example, a secondary topsheet can be an airlaid-tissue web made from hydrophilic cellulosic fibers and polyethylene powder, sometimes referred to as an airlaid STS. Or, a secondary topsheet can be a spunlace web. A spunlace web may be a hydroentangled fibrous structure with a basis weight between about 35 grams per square meter (gsm) and about 85 gsm. The spunlace web may comprise about 30% to about 60%, by weight, of cellulosic fibers, about 5% to about 30%, by weight, of non-cellulosic fibers, and about 30% to about 55%, by weight, of polyolefin-based binder fibers. Referring back to Figures 1 and 2, in one embodiment, the first substrate 102 comprises a secondary topsheet and the second substrate 104 comprises a topsheet. For example, the first substrate 102 may comprise a spunlace STS and the second substrate 104 may comprise a film-nonwoven composite topsheet, such as a polyethylene film-polyethylene nonwoven composite topsheet.

It is to be appreciated that the apparatuses and methods herein can be used to bond various types of substrates together. The substrates may comprise materials that can be deformed beyond their yield point by the compression in the nip of the apparatus. For example, in some embodiments the apparatus may be used to bond nonwoven substrates, such as for example, polypropylene nonwoven, polyethylene film, bi-component nonwoven or film, polyethylene terephthalate nonwoven or film. In some embodiments, the apparatuses and methods herein may be used to bond a substrate which includes a mixture of cellulosic fibers and polyethylene or polyethylene-polypropylene bicomponent fibers or particulate. In some embodiments, the substrates may have a basis weight of about 6 gsm to about 100 gsm. Other types of substrates can be sandwiched in between two layers of nonwovens or films.

The substrates may comprise any suitable woven, nonwoven, film, combination or laminate of any of the foregoing materials. Non-limiting examples of suitable substrates include cellulose, films, such as polymeric or thermoplastic films, foils, such as metallic foils (e.g. aluminum, brass, copper, and the like), webs comprising sustainable polymers, foams, fibrous nonwoven webs comprising synthetic fibers (e.g. TYVEK®), collagen films, chitosan films, rayon, cellophane, and the like. Suitable webs further include laminates or blends of these materials. Suitable films include both cast and blown. Exemplary thermoplastic films suitable for use as the second substrate are low density polyethylene ("LDPE"), linear low-density polyethylene ("LLDPE"), and blends of LLDPE and LDPE. Films may be apertured.

Substrates can also optionally include colorants, such as pigment, lake, toner, dye, ink or other agent used to impart a color to a material, to improve the visual appearance of a substrates or the resultant laminate. Suitable pigments herein include inorganic pigments, pearlescent pigments, interference pigments, and the like. Non-limiting examples of suitable pigments include talc, mica, magnesium carbonate, calcium carbonate, magnesium silicate, aluminum magnesium silicate, silica, titanium dioxide, zinc oxide, red iron oxide, yellow iron oxide, black iron oxide, carbon black, ultramarine, polyethylene powder, methacrylate powder, polystyrene powder, silk powder, crystalline cellulose, starch, titanated mica, iron oxide titanated mica, bismuth oxychloride, and the like. Suitable colored webs are described in US 2010/0233438 and US 2010/0233439.

Although the apparatuses and methods have been described in the context of the feminine hygiene article 5 shown in Figures 6-8, it is to be appreciated that the methods and apparatuses herein may be used to assemble and bond various substrates and/or elastic laminates that can be used with various process configurations and/or absorbent articles, such as for example, diapers or diaper pants.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention as defined in the appended claims. It is therefore intended to cover in the appended claims all such changes and modifications that are within the scope of this invention.

## Claims

1. An apparatus (100) for bonding substrates (102, 104) the apparatus (100) comprising:
a. an anvil roll (110); and
b. a bonding roll (106) comprising a base surface (120) and plurality of nubs (300) extending from the base surface (120), each of the plurality of nubs (300) comprising:
i. a sidewall (320);
ii. a bonding surface (310); and
iii. a shoulder (330) connecting the sidewall (320) to the bonding surface (310);
wherein the nub diameter is from 2 mm to 5 mm; and wherein the sidewall (320) is substantially perpendicular to the base surface (120);
**characterized in that** the shoulder (330) is chamfered at a 50 degree to 60 degree chamfer angle and **in that** a width of the shoulder (330) as measured in a plane parallel to the base surface (120) is from 0.2 mm to 0.6 mm.

2. The apparatus (100) according to claim 1, wherein the chamfered shoulder (330) comprises a chamfer angle of 55 degrees.

3. The apparatus (100) according to any one of the preceding claims, wherein the nubs (300) comprise a circular cross-sectional geometry.

4. The apparatus (100) according to any one of the preceding claims, wherein a ratio of the bonding surface (310) diameter to the nub (300) diameter is 0.75 or less.

5. The apparatus (100) according to any one of the preceding claims, wherein the bonding surface (310) has a diameter of from 1.0 mm to 3.0 mm.

6. The apparatus (100) according to any one of the preceding claims, wherein the nub (300) comprises a root radius (122) located between the sidewall (320) and the base surface (120).

## Patentansprüche

1. Apparat (100) zum Bonden von Substraten (102, 104), wobei der Apparat (100) Folgendes umfasst:
a. eine Ambosswalze (110); und
b. eine Bonding-Walze (106), umfassend eine Basisoberfläche (120) und eine Vielzahl von Noppen (300), die von der Basisoberfläche (120) ausgehen, wobei jede der Vielzahl von Noppen (300) umfasst:
i. eine Seitenwand (320);
ii. eine Bonding-Oberfläche (310); und
iii. eine Schulter (330), die die Seitenwand (320) mit der Bonding-Oberfläche (310) verbindet;
wobei der Noppendurchmesser von 2 mm bis 5 mm beträgt; und wobei die Seitenwand (320) im Wesentlichen lotrecht zu der Basisoberfläche (120) ist;
**dadurch gekennzeichnet, dass** die Schulter (330) bei einem Abschrägungswinkel von 50 Grad bis 60 Grad abgeschrägt ist, und dass eine Breite der Schulter (330) gemessen in einer zur Basisoberfläche (120) parallelen Ebene von 0,2 mm bis 0,6 mm ist.

2. Apparat (100) nach Anspruch 1, wobei die abgeschrägte Schulter (330) einen Abschrägungswinkel von 55 Grad umfasst.

3. Apparat (100) nach einem der vorstehenden Ansprüche, wobei die Noppen (300) eine kreisförmige Querschnittsgeometrie umfassen.

4. Apparat (100) nach einem der vorstehenden Ansprüche, wobei ein Verhältnis des Durchmessers der Bonding-Oberfläche (310) zu dem Durchmesser der Noppe (300) 0,75 oder weniger beträgt.

5. Apparat (100) nach einem der vorstehenden Ansprüche, wobei die Bonding-Oberfläche (310) einen Durchmesser von 1,0 mm bis 3,0 mm aufweist.

6. Apparat (100) nach einem der vorstehenden Ansprüche, wobei die Noppe (300) einen Wurzelradius (122) umfasst, der zwischen der Seitenwand (320) und der Basisoberfläche (120) angeordnet ist.

## Revendications

1. Appareil (100) pour lier des substrats (102, 104), l'appareil (100) comprenant :
a. un contre-rouleau (110) ; et
b. un rouleau de liaison (106) comprenant une surface de base (120) et une pluralité de protubérances (300) s'étendant à partir de la surface de base (120), chacune parmi la pluralité de protubérances (300) comprenant :
i. une paroi latérale (320) ;
ii. une surface de liaison (310) ; et
iii. un épaulement (330) reliant la paroi latérale (320) à la surface de liaison (310) ;
dans lequel le diamètre de protubérance va de 2 mm à 5 mm ; et dans lequel la paroi latérale (320) est essentiellement perpendiculaire à la surface de base (120) ;
**caractérisé en ce que** l'épaulement (330) est chanfreiné à un angle de chanfrein de 50 degrés à 60 degrés et **en ce qu'**une largeur de l'épaulement (330) telle que mesurée dans un plan parallèle à la surface de base (120) va de 0,2 mm à 0,6 mm.

2. Appareil (100) selon la revendication 1, dans lequel l'épaulement chanfreiné (330) comprend un angle de chanfrein de 55 degrés.

3. Appareil (100) selon l'une quelconque des revendications précédentes, dans lequel les protubérances (300) comprennent une géométrie en coupe circulaire.

4. Appareil (100) selon l'une quelconque des revendications précédentes, dans lequel un rapport du diamètre de la surface de liaison (310) au diamètre de la protubérance (300) vaut 0,75 ou moins.

5. Appareil (100) selon l'une quelconque des revendications précédentes, dans lequel la surface de liaison (310) a un diamètre allant de 1,0 mm à 3,0 mm.

6. Appareil (100) selon l'une quelconque des revendications précédentes, dans lequel la protubérance (300) comprend un rayon de racine (122) situé entre la paroi latérale (320) et la surface de base (120).
